# EUROPÄISCHE PATENTSCHRIFT

(11) **EP 0 579 212 B1**
(45) Veröffentlichungstag und Bekanntmachung des Hinweises auf die Patenterteilung: **15.04.1998**
(21) Anmeldenummer: 93111315.3
(22) Anmeldetag: 14.07.1993
(51) Int. Cl.: C07D 233/68, C07D 233/70

(54) **Verfahren zur Herstellung von 2-substituierten 5-Chlorimidazol-4-carbaldehyden**
Process of preparation of 2-substituted 5-chlorimidazol-4-carbaldehydes
Procédé pour la préparation de 5-chloroimidazol-4-carbaldéhydes 2-substituées

(30) Priorität: 16.07.1992 CH 2239/92
(43) Veröffentlichungstag der Anmeldung: 19.01.1994
(73) Patentinhaber: LONZA AG, CH-3945 Gampel/Wallis (CH)
(72) Erfinder: Gosteli, Jacques, Dr., Basel (CH); Griffiths, Gareth, Dr., Visp (Kanton Wallis) (CH); Imwinkelried, René, Dr., Brig-Glis (Kanton Wallis) (CH)
(74) Vertreter: Weinhold, Peter, Dr.

(56) Entgegenhaltungen:
- EP-A- 28 834
- EP-A- 430 709
- EP-A- 450 566
- DE-A- 2 804 435
- CHEMICAL ABSTRACTS, vol. 116, no. 25, 22. Juni 1992, Columbus, Ohio, US; abstract no. 255614j, Seite 792 ;Spalte 2 ;
- TETRAHEDRON LETTERS Bd. 31, Nr. 36, 1990, GREAT BRITAIN Seiten 5227 - 5230 PAUL R. GILES ET AL. 'Oxidative formylation and chloromethylation as alternative pathways to chloroformylation in the reaction of O- and S-heterocyclic ketones with Dimethylformamide-Phosphorus oxychloride'
- THE JOURNAL OF ORGANIC CHEMISTRY Bd. 52, Nr. 13, 26. Juni 1987, COLUMBUS OHIO Seiten 2726 - 2730 ALAN R. KATRITZKY ET AL. 'Reactions of 2-Cyclohexen-1-ones and Cyclohexane 1,3-diones with Chloromethylene Iminium Salts'

## Beschreibung

Die Erfindung beinhaltet ein neues Verfahren zur Herstellung von 2-substituierten 5-Chlorimidazol-4-carbaldehyden der allg. Formel 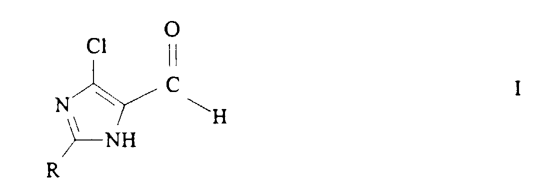 worin R eine Alkylgruppe, eine Cycloalkylgruppe, eine Benzylgruppe, eine Phenylgruppe oder eine Arylgruppe bedeutet. Unter einer Alkylgruppe werden geradkettige oder verzweigte C₁-C₆-Alkylgruppen, wie z. B. Methyl-, Ethyl-, Propyl-, Isopropyl-, n-Butyl, sec. Butyl-, tert. Butyl-, Pentyl- oder Hexylgruppen verstanden. Bevorzugte Alkylgruppe ist die n-Butylgruppe.
Als Vertreter von Cycloalkylgruppen seien die Cyclopropyl-, die Cyclobutyl-, die Cyclopentyloder die Cyclohexylgruppe genannt.
Sowohl die Benzyl- als auch die Phenylgruppe kann Substituenten wie die genannten Alkylgruppen, Halogenatome, Nitrogruppen oder Aminogruppen enthalten.
Die 2-substituierten 5-Chlorimidazol-4-carbaldehyde der allg. Formel I sind wichtige Ausgangsprodukte zur Herstellung von blutdrucksenkenden Pharmazeutika (US-PS 4 355 040) oder von herbizidwirksamen Verbindungen (DE-OS 2804435).

Es sind mehrere Wege zur Herstellung der genannten Verbindungen gemäss allg. Formel I bekannt.

So beschreibt die US-PS 4 355 040 ein Verfahren nach welchem 2-Amino-3,3-dichloracrylnitril mit einem Aldehyd zum entsprechenden Azomethinzwischenprodukt und weiter mit einem Halogenwasserstoffund Wasser zum 2-substituierten-5-halogenimidazol-4-carbaldehyd umgesetzt wird. Experimentielle Angaben fehlen in der genannten Patentschrift. Ein grosser Nachteil der Synthese besteht aber darin, dass das eingesetzte 2-Amino-3,3-dichloracrylnitril zunächst ausgehend von Dichloracetonitril durch dessen Umsetzung mit Blausäure/Natriumcyanid hergestellt werden muss. Die äusserst toxischen Reaktionsteilnehmer, und die damit verbundenen sicherheitstechnischen Massnahmen , die bereits für die Bereitstellung des Ausgangsproduktes notwendig sind, machen das Gesamtverfahren industriell untauglich.

Die US-PS 4 355 040 offenbart in einer weiteren Variante ein 3-Stufen Verfahren, worin in einer ersten Stufe ein Amidinhydrochlorid mit Dihydroxyaceton mit hohem NH₃-Druck ringgeschlossen wird, der Imidazolalkohol halogeniert wird und schliesslich zum Aldehyd oxidiert wird.
Es hat sich gezeigt, dass für die Ringschlussreaktion Drucke von über 20 bar notwendig sind.
Die Oxidation des Alkohols funktioniert gemäss US-PS 4 355 040 in Gegenwart von Chromoxid.
Es ist offensichtlich, dass eine Oxidation mit Schwermetalloxiden, die in der Regel nicht rezirkulierbar sind nach heutigen ökologischen Gesichtspunkten nicht mehr verantwortbar ist.

Es bestand folglich die Aufgabe, ein Verfahren zu entwickeln, das die genannten Nachteile nicht aufweist.

Die Aufgabe konnte gelöst werden mit einem neuen Verfahren nach Anspruch 1.

Erfindungsgemäss wird ein 2-substituiertes 3,5-Dihydroimidazolin-4-on der allg. Formel 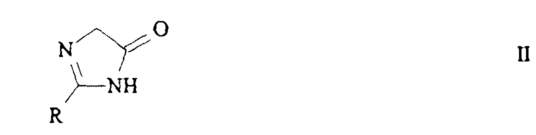 worin R die genannte Bedeutung mit Phosphoroxychlorid in Gegenwart von N,N-Dimethylformamid zum gewünschten 2-substituierten 5-Chlorimidazol-4-carbaldehyd umgesetzt.
Die 2-substituierten 3,5-Dihydroimidazolin-4-one der allg. Formel II sind im allgemeinen auf bekannte Weise, gemäss
R. Jacquier et al., Bull. Soc. Chim. France 1971, S. 1040 f. durch Umsetzung eines substituierten Imidsäurealkylesters der allg. Formel 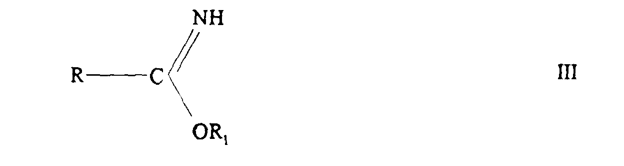 worin R die genannte Bedeutung hat und R₁ eine Niederalkylgruppe mit 1 bis 4 C-Atomen bedeutet mit einem Glycinniederalkylester zugänglich.

Besonders bevorzugt wird im Rahmen des erfindungsgemässen Verfahrens Glycinethylester mit Pentanimidsäurethylester (allg. Formel III mit R = n-Butyl und R₁ = Ethyl) das 2-n-Butyl-3,5-dihydroimidazolin4-on (allg. Formel II mit R = n-Butyl) synthetisiert. Diese Verbindung ist nicht literaturbekannt und daher ebenfalls Bestandteil der Erfindung.

Für die Umsetzung zum gewünschten 2-substituierten 5-Chlorimidazol4-carbaldehyd der allg. Formel I wird zweckmässig so vorgegangen, dass zunächst Phosphoroxychlorid und N,N-Dimethylformamid im Molverhältnis 2 zu 1 bis 4 zu 1 vorgelegt werden. Das entsprechende 2-substituierte 3,5-Dihydroimidazolin-4-on wird zweckmässig darauf dem Reaktionsgemisch zugeben, worauf die Umsetzung vorteilhaft in einem Temperaturbereich zwischen 50°C und 80°C erfolgt.

Die Aufarbeitung des Endproduktes kann auf fachmännische Weise erfolgen.

Bevorzugt wird nach dem erfindungsgemässen Verfahren ausgehend von 2-n-Butyl-3,5-dihydroimidazolin-4-on das 2-n-Butyl-5-chlorimidazol-4-carbaldehyd hergestellt.

### Beispiele:

### 2-n-Butyl-3,5-dihydroimidazolin-4-on

Ein Gemisch von Glycinethylester (24,5 g, 221 mmol) und Pentanimidsäureethylester (34,5 g, 254 mmol) wurde 36 h bei -18°C stehengelassen. Das ausgefallene Produkt wurde abfiltriert, mit eiskaltem Diethylether (70 ml) gewaschen und getrocknet.
Ausbeute 10,51 g (34%).
Smp. 79,5 - 80,5°C
- ¹H-NMR: (CDCl₃, 300 MHz) δ in ppm: 9,3, 1H br; 4,1, 2H, m; 2,48, 2H, t; 1,68, 2H, m; 1,45, 2H, m; 0,95, 3H, t

### 2-n-Butyl-5-chlorimidazol-4-carbaldehyd

Zu POCl₃ (30,65 g, 200 mmol) bei ca. 20°C wurde N,N-Dimethylformamid (3,65 g, 50 mmol) zugetropft. Das rötliche Gemisch wurde 15 Min. bei 20 °C gerührt. 2-n-Butyl-3,5-dihydroimidazolin-4-on (1,40g, 10 mmol) wurde dann portionenweise zugegeben und das Gemisch wurde 1 h bei 80°C erhitzt. Überschüssiges POCl₃ wurde am Rotationsverdampfer entfernt und der ölige Rückstand wurde aufEis gegossen. Der pH wurde mit gesättigter NaHCO₃-Lsg. auf 7 gestellt und das Gemisch wurde dreimal mit je 150 ml Essigsäureethylester extrahiert. Die vereinigten organischen Phasen wurden getrocknet (MgSO₄), filtriert und evaporiert. Reinigung des Rohproduktes mittels Säulenchromatographie auf Kieselgel ergab 2-n-Butyl-5-chlorimidazol-4-carbaldehyd.
Ausbeute 0,25 g, 14 %, Gehalt ca 95% (¹H-NMR).

## Patentansprüche

1. Verfahren zur Herstellung von 2-substituierten 5-Chlorimidazol-4-carbaldehyden der allgemeinen Formel 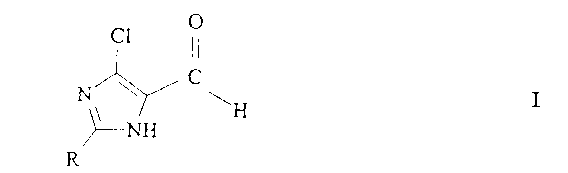 worin R eine Alkylgruppe, eine Cycloalkylgruppe, eine Benzylgruppe, eine Phenylgruppe oder eine Arylgruppe bedeutet, dadurch gekennzeichnet, daß ein 2-substituiertes 3,5-Dihydroimidazolin-4-on der allgemeinen Formel 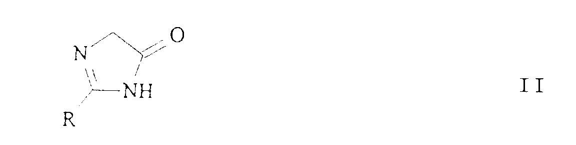 worin R die genannte Bedeutung hat, mit Phosphoroxychlorid in Gegenwart von N,N-Dimethylformamid im Molverhältnis Phosphoroxychlorid zu N,N-Dimethylformamid von 2 zu 1 bis 4 zu 1 zum Endprodukt umgesetzt wird.

2. Verfahren nach Anspruch 1, dadurch gekennzeichnet, daß als 2-substituiertes 3,5-Dihydroimidazolin-4-on der allgemeinen Formel II das 2-n-Butylderivat mit R = n-Butyl eingesetzt wird.

3. Verfahren nach einem der Ansprüche 1 oder 2, dadurch gekennzeichnet, daß die Umsetzung bei einer Temperatur zwischen 50°C und 80°C erfolgt.

4. 2-n-Butyl-3,5-dihydroimidazolin-4-on.

## Claims

1. A method for producing 2-substituted 5-chloroimidazole-4-carbaldehydes having the general formula 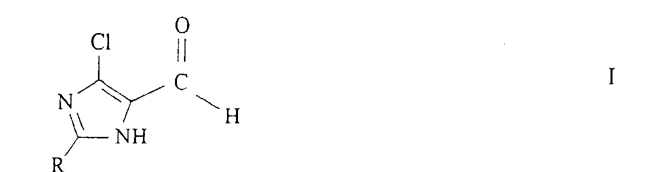 wherein R represents an alkyl group, a cycloalkyl group, a benzyl group, a phenyl group or an aryl group, characterized in that a 2-substituted 3,5-dihydroimidazolin-4-one having the general formula 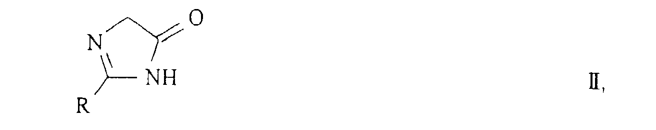 wherein R has the specified meaning, is reacted with phosphorus oxychloride in the presence of N,N-dimethylformamide in a molar ratio phosphorus oxychloride:N,N-dimethlformamide of 2:1 to 4:1 to form the final product.

2. The method of claim 1, characterized in that the 2-n-butyl derivative wherein R = n-butyl is employed as a 2-substituted 3,5-dihydroimidazolin-4-one of general formula II.

3. The method of one of claims 1 or 2, characterized in that the reaction is carried out at a temperature between 50°C and 80°C.

4. 2-n-butyl-3,5-dihydroimidazolin-4-one.

## Revendications

1. Procédé pour la préparation de 5-chlorimidazol-4-carbaldéhydes 2-substitués de la formule générale : 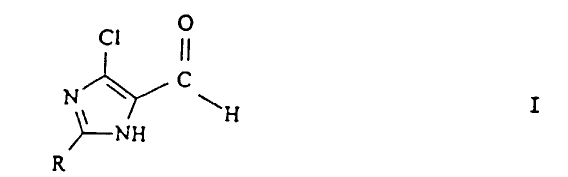 dans laquelle R signifie un groupe alkyle, un groupe cycloalkyle, un groupe benzyle, un groupe phényle ou un groupe aryle, caractérisé en ce qu'une 3,5-dihydro-imidazolin-4-one 2-substituée de la formule générale 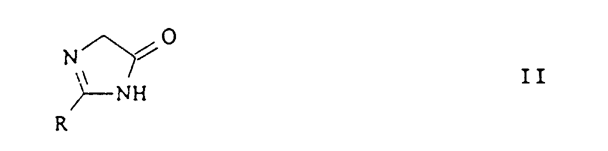 dans laquelle R a la signification indiquée, est mise en réaction avec de l'oxychlorure de phosphore en présence de N,N-diméthylformamide dans un rapport molaire entre l'oxychlorure de phosphore et le N,N-diméthylformamide de 2 à 1 jusqu'à 4 à 1 pour obtenir le produit final.

2. Procédé selon la revendication 1, caractérisé en ce qu'en tant que 3,5-dihydro-imidazolin-4-one 2-substituée de la formule générale II, on met en oeuvre le dérivé de 2-n-butyle avec R = n-butyle.

3. Procédé selon l'une des revendications 1 ou 2, caractérisé en ce que la réaction est conduite à une température entre 50 et 80°C.

4. 2-n-butyl-3,5-dihydro-imidazolin-4-one.
